# EUROPÄISCHE PATENTSCHRIFT

(11) **EP 0 613 482 B1**
(45) Veröffentlichungstag und Bekanntmachung des Hinweises auf die Patenterteilung: **04.06.1997**
(21) Anmeldenummer: 92923238.7
(22) Anmeldetag: 06.11.1992
(51) Int. Cl.: C07H 15/04

(54) **VERFAHREN ZUR HERSTELLUNG VON ALKYL- UND/ODER ALKENYLOLIGOGLYKOSIDEN**
METHOD OF PREPARING ALKYL AND/OR ALKENYL OLIGOGLYCOSIDES
PROCEDE DE FABRICATION D'ALKYL- ET/OU D'ALCENYLOLIGOGLUCOSIDES

(30) Priorität: 15.11.1991 DE 4137636
(43) Veröffentlichungstag der Anmeldung: 07.09.1994
(73) Patentinhaber: Henkel Kommanditgesellschaft auf Aktien, 40191 Düsseldorf (DE)
(72) Erfinder: WEUTHEN, Manfred, D-5650 Solingen 11 (DE); HILL, Karlheinz, D-4006 Erkrath (DE); SCHULZ, Paul, D-5600 Wuppertal 1 (DE)
(86) Internationale Anmeldenummer: EP9202552
(87) Internationale Veröffentlichungsnummer: WO9310132

(56) Entgegenhaltungen:
- EP-A- 0 415 192
- EP-A- 0 415 192
- AU-B- 538 363
- CHEMICAL ABSTRACTS, vol. 91, no. 19, 5. November 1979, Columbus, Ohio, US; abstract no. 157440v, H. HAGIWARA ET AL. 'Alkylphenols' Seite 612 ;Spalte 1 ;

## Beschreibung

### Gebiet der Erfindung

Die Erfindung betrifft ein Verfahren zur Herstellung von Alkyl- und/oder Alkenyloligoglykosiden durch Acetalisierung von Glykosen mit Fettalkoholen bei erhöhten Temperaturen in Gegenwart von Sulfomonocarbonsäuren als sauren Katalysatoren, Entfernen des Reaktionswassers und anschließende Aufarbeitung.

### Stand der Technik

Oberflächenaktive Alkyloligoglykoside, insbesondere Alkyloligoglucoside, sind seit langem als wertvolle Rohstoffe für die Herstellung von Waschmitteln und kosmetischen Produkten bekannt. Ihre Herstellung erfolgt üblicherweise entweder durch direkte Acetalisierung von Glykose mit Fettalkoholen ("Direktsynthese") oder über die Zwischenstufe der Butylglykoside, die einer Umacetalisierung mit Fettalkoholen unterworfen werden ("Butanol-Route"), wobei das Reaktionswasser sowie gegebenenfalls Butanol kontinuierlich aus dem Gleichgewicht entfernt wird. Die Aufarbeitung der rohen Alkyloligoglykoside umfaßt die Neutralisation der Produkte, das Abtrennen des überschüssigen Fettalkohols sowie gegebenenfalls Bleiche und Anpastung. Im Hinblick auf den umfangreichen Stand der Technik sei stellvertretend auf die Druckschriften **EP 0 319 616 A1** und **WO 89/00923** verwiesen.

Die Acetalisierung von Glykosen bzw. die Umacetalisierung von Glykosiden mit Fettalkoholen erfolgt stets in Gegenwart saurer Katalysatoren. Typische Beispiele hierfür sind Schwefelsäure **[US 3,974,138]**, Alkylbenzolsulfonsäure **[US 5,003, 057]**, p-Toluolsulfonsäure **[EP 0 301 298 A1]** oder Sulfobernsteinsäure **[EP 0 415 192 A1]**.

Der Einsatz der genannten Katalysatoren ist jedoch mit technischen Schwierigkeiten verbunden. Bei der Verwendung von Schwefelsäure kann es zu einer partiellen Verkokung der Glykose kommen, Alkylbenzolsulfonsäure zeigt eine unbefriedigende katalytische Aktivität, die Verwendung von p-Toluolsulfonsäure ist mit unvollständigen Ausbeuten verbünden und Sulfobernsteinsäure ist nicht zufriedenstellend biologisch abbaubar.

Die Aufgabe der Erfindung bestand somit darin, neue Katalysatoren für die Acetalisierung von Glykosen bzw. für die Umacetalisierung von Glykosiden mit Fettalkoholen zu entwikkeln, die frei von den geschilderten Nachteilen sind.

### Beschreibung der Erfindung

Gegenstand der Erfindung ist ein Verfahren zur Herstellung von Alkyl- und/oder Alkenyloligoglykosiden durch Acetalisierung von Glykose mit Fettalkoholen bei erhöhter Temperatur in Gegenwart von sauren Katalysatoren, Entfernen des Reaktionswasers und Aufarbeitung des Reaktionsgemisches, das sich dadurch auszeichnet, daß man die Umsetzung in Gegenwart von Sulfomonocarbonsäuren mit 2 bis 8 Kohlenstoffatomen, deren Carbonsäureanhydride und/oder deren gemischten cyclischen Carbonsäure-Sulfonsäureanhydriden als saure Katalysatoren durchführt.

Überraschenderweise wurde gefunden, daß die Verwendung von Sulfomonocarbonsäuren in der Acetalisierung nicht nur zu hohen Ausbeuten und geringen Mengen an unerwünschten Nebenprodukten, insbesondere Polyglykose, führt, sondern auch mit kurzen Reaktionszeiten und damit einer vorteilhaften Anlagenauslastung verbunden ist. Des weiteren lassen sich die Sulfocarbonsäuren problemlos in Fettalkohole einarbeiten und sind somit leicht dosierbar. Schließlich zeichnen sich die Katalysatoren durch eine hohe ökologische und toxikologische Verträglichkeit aus.

**Sulfomocarbonsäuren** stellen bekannte Stoffe dar, die nach den einschlägigen Methoden der präparativen organischen Chemie erhalten werden können. Eine Möglichkeit besteht beispielsweise darin, kurzkettige Hydroxycarbonsäuren zu sulfatieren und anschließend mit Bisulfit umzusetzen. Des weiteren ist es ebenfalls möglich, kurzkettige aliphatische Carbonsäuren oder Benzoesäure in inerten Lösungsmitteln vorzulegen und mit Schwefelsäure, Chlorsulfonsäure oder Schwefeltrioxid zu sulfonieren **[Rec. trav.chim. 43, 297, 420 (1924); 71, 814 (1952)]**.

Sulfomonocarbonsäuren, die als Katalysatoren im Sinne der Erfindung in Betracht kommen, weisen 2 bis 8, vorzugsweise 2 bis 4 Kohlenstoffatome auf. Typische Beispiele sind Sulfoessigsäure, Sulfopropionsäure, Sulfobuttersäure, Sulfovaleriansäure, Sulfocapronsäure, Sulfocaprylsäure, Sulfocaprinsäure oder Sulfobenzoesäure. Die Sulfomonocarbonsäuren können auch in Form ihrer Carbonsäureanhydride oder der gemischten cyclischen Sulfonsäure-Carbonsäure-Anhydride eingesetzt werden. Beispiele hierfür sind Sulfoessigsäureanhydrid sowie das gemischte cyclische Sulfonsäure-Carbonsäure-Anhydrid der Sulfopropionsäure.

Die sauren Katalysatoren können in Mengen von 0,01 bis 2, vorzugsweise 0,1 bis 0,5 Gew.-% - bezogen auf die Glykose - eingesetzt werden.

Unter **Glykosen**, die als Ausgangsstoffe für die Herstellung von Alkyl- und/oder Alkenyloligoglykosiden im Sinne des erfindungsgemäßen Verfahrens in Betracht kommen, sind Aldosen bzw. auch Ketosen im weitesten Sinne zu verstehen. Typische Beispiele sind Glucose, Fructose, Mannose, Galactose, Talose, Gulose, Allose, Altrose, Idose, Arabinose, Xylose, Lyxose und Ribose. Vorzugsweise werden wegen der besseren Reaktionsfähigkeit die Aldosen verwendet. Unter den Aldosen kommt wegen ihrer leichten Zugänglichkeit und Verfügbarkeit in technischen Mengen insbesondere die Glucose in Betracht. Die nach dem Verfahren der Erfindung besonders bevorzugt hergestellten Alkyl- und/oder Alkenyloligoglykoside sind deshalb die Alkyl und/oder Alkenyloligoglucoside.

Handelsübliche Glucose enthält in der Regel 1 Mol Kristallwasser. Diese kristallwasserhaltige Glucose kann ohne weiteres verwendet werden. Es hat es sich jedoch als zweckmäßig erwiesen, das Kristallwasser zusätzlich, und zwar vor dem Inkontaktbringen mit dem Katalysator aus dem Reaktionsmilieu durch thermische Maßnahmen zu entfernen. Nachdem aber auch wasserfreie Glucose in großen Mengen am Markt erhältlich ist, wird diese bevorzugt in Form eines feinteiligen Pulvers eingesetzt.

Unter **Fettalkoholen** sind primäre, aliphatische Alkohole zu verstehen, die der Formel (I) folgen,

R¹-OH (I)

in der R¹ für einen linearen oder verzweigten Alkyl- oder Alkenylrest mit 4 bis 22 Kohlenstoffatomen steht.

Typische Beispiele sind n-Butanol, i-Butanol, Capronalkohol, Caprylalkohol, Caprinalkohol, Myristylalkohol, Laurylalkohol, Cetylalkohol, Stearylalkohol, Oleylalkohol, Elaidylalkohol, Petroselinylalkohol, Arachylalkohol, Behenylalkohol und Erucylalkohol sowie deren Gemische.

Wie in der Fettchemie üblich, können die Fettalkohole auch in Form technischer Gemische vorliegen, wie sie z. B. durch Hochdruckhydrierung von Methylestern auf Basis pflanzlicher oder tierischer Öle und Fette, beispielsweise Palmöl, Palmkernöl, Kokosöl, Rüböl, Sonnenblumenöl oder Rindertalg erhalten werden. Eine weitere Gruppe technischer geeigneter primärer Alkohole stellen die Oxoalkohole des angegebenen Kohlenstoffzahlbereichs dar, die durch Hydrierung von Aldehyden aus der Roelen'schen Oxoreaktion gewonnen werden und einen Anteil von 5 bis 25 Gew.-% verzweigter Species enthalten können. Bevorzugt ist der Einsatz von technischen Kokosfettalkoholschnitten mit 12 bis 18, vorzugsweise 12 bis 14 Kohlenstoffatomen.

Das molare Einsatzverhältnis von Glykose zu Fettalkohol kann dabei 1 : 1,5 bis 1 : 10, vorzugsweise 1 : 3 bis 1 : 5 betragen.

Die Sulfomonocarbonsäuren können mit der Glykose und dem Fettalkohol unter Rühren in Kontakt gebracht werden. Dabei ist es unkritisch, ob die Vermischung vor dem Aufheizen stattfindet oder ob der Katalysator zu der auf Reaktionstemperatur erhitzten Mischung aus Glykose und Fettalkohol zudosiert wird. In einer bevorzugten Ausführungsform des erfindungsgemäßen Verfahrens werden die Sulfomonocarbonsäuren in einem Teil des Fettalkohols präformiert, d.h. bei Temperaturen von 20 bis 40°C unter Rühren gelöst, und anschließend der Reaktionsmischung aus Glykose und restlichem Fettalkohol bei der Reaktionstemperatur zugesetzt. In manchen Fällen, insbesondere bei der Herstellung von kurzkettigen Alkyl- und/oder Alkenylglykosiden, kann es jedoch auch vorteilhaft sein, wenn der vorgelegte Fettalkohol und das Lösungsmittel für den sauren Katalysator verschieden sind. Beide Ausführungsformen haben sich im Hinblick auf eine leichte Löslichkeit des Katalysators im Reaktionsansatz als optimal erwiesen.

Die eigentliche Acetalisierung kann bei Temperaturen von 80 bis 130, vorzugsweise 90 bis 120°C erfolgen, wobei es sich als vorteilhaft erwiesen hat, die Reaktion unter einem vermindertem Druck von 1 bis 100 mbar durchzuführen.

Zur Verlagerung des Gleichgewichtes empfiehlt es sich ferner, das gebildete Reaktionswasser sowie Wasser, das mit den Einsatzstoffen eintragen wurde, kontinuierlich beispielsweise mit Hilfe einer Destillationsvorrichtung zu entfernen. Werden kurzkettige Fettalkohole eingesetzt, die mit Wasser leicht flüchtige Azeotrope bilden, empfiehlt es sich, die wäßrigen Alkohole nach der Destillation aufzuarbeiten und das Wertprodukt wieder zurückzuführen.

Das Ende der Reaktion kann dadurch erkannt werden, daß kein weiteres Reaktionswasser gebildet und abdestilliert wird. Um sicherzustellen, daß sich die Glykose möglichst vollständig, d. h. zu mindestens 99 Gew.-% - bezogen auf die Ausgangsmenge - umgesetzt hat, empfiehlt es sich, die Reaktionsmischung bei der Reaktionstemperatur einer Nachreaktion zu unterwerfen und den Restzuckergehalt beispielsweise durch die FEHLING-Probe zu überprüfen.

Die weitere Aufarbeitung der rohen Alkyl- und/oder Alkenyloligoglykoside kann in an sich bekannter Weise erfolgen. Zunächst werden die sauren Reaktionsprodukte mit Alkali- und/ oder Erdalkalioxiden, -hydroxiden oder -carbonaten neutralisiert und anschließend der überschüssige Fettalkohol destillativ, vorzugsweise unter Verwendung eines Dünnschichtverdampfers bei einer Temperatur von 160 bis 240°C und einem verminderten Druck von 1 bis 10 mbar abgetrennt. In den meisten Fällen ist es empfehlenswert, die resultierenden Alkyl- und/oder Alkenyloligoglykoside einer Bleiche zu unterziehen und gegebenenfalls gleichzeitig mit Wasser anzupasten.

### Gewerbliche Anwendbarkeit

Die nach dem erfindungsgemnäßen Verfahren hergestellten Alkyl- und/oder Alkenyloligoglykoside zeichnen sich durch einen niedrigen Polyglykosegehalt aus und weisen oberflächenaktive Eigenschaften auf. Sie eignen sich daher zur Herstellung von Wasch-, Spül- und Reinigungsmitteln sowie Produkten zur Haar- und Körperpflege, in denen sie in Mengen von 0,1 bis 50, vorzugsweise 1 bis 25 Gew.-% - bezogen auf die Mittel - enthalten sein können.

Die folgenden Beispiele sollen den Gegenstand der Erfindung näher erläutern, ohne ihn darauf einzuschränken.

### Beispiele

**Allgemeine Versuchsvorschrift.** In einem 2-1-Dreihalskolben mit Rührer, Destillationsaufsatz und Innenthermometer wurden 180 g (1 Mol) wasserfreie Glucose und 870 g (4,5 mol) C_{12/14}-Kokosfettalkohol (Lorol^{(R)} S, Hydroxylzahl 290, Fa.Henkel KGaA, Düsseldorf, FRG) vorgelegt und unter einem verminderten Druck von ca. 20 mbar auf 110°C erhitzt. Anschließend wurde die Reaktionsmischung mit 0,1 bis 0,5 Gew.-% - bezogen auf die Glucose - Katalysator in Form einer 5 gew.-%igen Lösung in Kokosfettalkohol versetzt. Zur Verlagerung des Gleichgewichtes wurde das Reaktionswasser kontinuierlich abdestilliert und die Reaktion abgebrochen, nachdem die Wasserabscheidung beendet war und der Restgehalt an nicht umgesetzter Glucose in der Mischung weniger als 1 Gew.-% - bezogen auf die Ausgangsmenge - betrug. Danach wurde die Reaktionsmischung mit Magnesiumoxid neutralisiert und der überschüssige Kokosfettalkohol unter vermindertem Druck (ca. 1 mbar) und einer Temperatur von 180°C mit Hilfe eines Dünnschichtverdampfers abgetrennt.

Angaben zu den Versuchsansätzen sowie die Kenndaten der Produkte sind in Tab.1 zusammengefaßt.

**Tab.1:**

| Acetalisierung von Glucose mit Kokosfettalkohol Prozentangaben als Gew.-% | | | | | | |
|---|---|---|---|---|---|---|
| Bsp. | Kat. | c (Kat) % | t min | A g | c (Pol) % | DP |
| 1 | A1 | 0,1 | 390 | 288 | 5,2 | 1,37 |
| 2 | A2 | 0,1 | 330 | 285 | 5,5 | 1,34 |
| V1 | B | 0,1 | 900 | 286 | 5,7 | 1,33 |
| V2 | B | 0,5 | 420 | 286 | 6,1 | 1,33 |
| Legende: Kat. = Katalysator c(Kat) = Katalysatorkonzentration t = Reaktionszeit A = Ausbeute c(Pol) = Gehalt an Polyglucose DP = Durchschnittlicher Polymerisationsgrad A1 = Sulfoessigsäure A2 = Gemischtes cyclisches Sulfonsäure-Carbonsäureanhydrid der Sulfopropionsäure B = Dodecylbenzolsulfonsäure | | | | | | |

## Patentansprüche

1. Verfahren zur Herstellung von Alkyl- und/oder Alkenyloligoglykosiden durch Acetalisierung von Glykose mit Fettalkoholen bei erhöhter Temperatur in Gegenwart von sauren Katalysatoren, Entfernen des Reaktionswasers und Aufarbeitung des Reaktionsgemisches, **dadurch gekennzeichnet,** daß man die Umsetzung in Gegenwart von Sulfomonocarbonsäuren mit 2 bis 8 Kohlenstoffatomen, deren Carbonsäureanhydriden und/oder gemischten cyclischen Sulfonsäure-Carbonsäureanhydriden als saure Katalysatoren durchführt.

2. Verfahren nach Anspruch 1, **dadurch gekennzeichnet,** daß man als saure Katalysatoren Sulfoessigsäure, Sulfopropionsäure, Sulfobuttersäure oder Sulfobenzoesäure einsetzt.

3. Verfahren nach den Ansprüchen 1 und 2, **dadurch gekennzeichnet,** daß man die sauren Katalysatoren in Mengen von 0,01 bis 2 Gew.-% - bezogen auf die Glykose - einsetzt.

4. Verfahren nach mindestens einem der Ansprüche 1 bis 3, **dadurch gekennzeichnet,** daß man als Glykose Glucose einsetzt.

5. Verfahren nach mindestens einem der Ansprüche 1 bis 4, **dadurch gekennzeichnet,** daß man Fettalkohole der Formel (I) einsetzt,
R¹-OH (I)
in der R¹ für einen linearen oder verzweigten Alkyl- oder Alkenylrest mit 4 bis 22 Kohlenstoffatomen steht.

## Claims

1. A process for the production of alkyl and/or alkenyl oligoglycosides by acetalization of glycose with fatty alcohols at elevated temperature in the presence of acidic catalysts, removal of the water of reaction and working up of the reaction mixture, **characterized in tha**t the reaction is carried out in the presence of sulfomonocarboxylic acids containing 2 to 8 carbon atoms, carboxylic anhydrides thereof and/or mixed cyclic sulfonic/carboxylic anhydrides thereof as acidic catalysts.

2. A process as claimed in claim 1, **characterized in that** sulfoacetic acid, sulfopropionic acid, sulfobutyric acid or sulfobenzoic acid is used as the acidic catalyst.

3. A process as claimed in claims 1 and 2, **characterized in that** the acidic catalysts are used in quantities of 0.01 to 2% by weight, based on the glycose.

4. A process as claimed in at least one of claims 1 to 3, **characterized in that** glucose is used as the glycose.

5. A process as claimed in at least one of claims 1 to 4, **characterized in that** fatty alcohols corresponding to formula (I)
R¹-OH (I)
in which R¹ is a linear or branched alkyl or alkenyl radical containing 4 to 22 carbon atoms are used.

## Revendications

1. Procédé d'obtention d'alkyl et/ou d'alcényloligoglycosides par acétalisation de glycose avec des alcools gras, à température accrue, en présence de catalyseurs acides, élimination de l'eau réactionnelle, et traitement du mélange de réaction,
caractérisé en ce qu'
on effectue la réaction en présence d'acides sulfomonocarboxyliques ayant de 2 à 8 atomes de carbone, de leurs anhydrides d'acide carboxylique et/ou des anhydrides mixtes cycliques d'acide sulfonique/acide carboxylique, en tant que catalyseur acide.

2. Procédé selon la revendication 1,
caractérisé en ce qu'
on met en oeuvre comme catalyseurs acides de l'acide sulfoacétique, de l'acide sulfopropionique, de l'acide sulfobutyrique, ou de l'acide sulfobenzoïque.

3. Procédé selon les revendications 1 et 2,
caractérisé en ce qu'
on met en oeuvre les catalyseurs acides en quantités allant de 0,01 à 2 % en poids, rapporté au glycose.

4. Procédé selon au moins une des revendications 1 à 3,
caractérisé en ce qu'
on met en oeuvre comme glycose, du glucose.

5. Procédé selon au moins une des revendications 1 à 4,
caractérisé en ce qu'
on met en oeuvre des alcools gras de formule (I),
R¹-OH (I)
dans laquelle R¹ représente un radical alkyl ou alcényle linéaire ou ramifié ayant de 4 à 22 atomes de carbone.
